# EUROPEAN PATENT APPLICATION

(11) **EP 4 722 191 A1**
(43) Date of publication of application: **08.04.2026**
(21) Application number: 24383065.0
(22) Date of filing: 02.10.2024
(51) Int. Cl.: C07C 231/02, C07C 233/25

(54) **SYNTHESIS OF PARACETAMOL VIA SPRAY-DRYING**

(71) Applicant: Fundació Institut Català de Nanociència i Nanotecnologia (ICN2), 08193 Bellaterra (ES); Institució Catalana De Recerca I Estudis Avançats (ICREA), 08010 Barcelona (ES)
(72) Inventor: MASPOCH COMAMALA, Daniel, 08197 Valldoreix (ES); IMAZ GABILONDO, Inhar, 08240 Manresa (ES); PENA POZO, Gerard, 08210 Barberà del Vallès (ES); ALBALAD ALCALÁ, Jorge, 08100 Mollet del Vallès (ES)
(74) Representative: ZBM Patents - Zea, Barlocci & Markvardsen

(57) **Abstract**

The present invention relates to the field of chemical synthesis of compounds, such as pharmaceutical compounds, and refers to a process to prepare amides by performing the synthesis of paracetamol via spray-drying.

## Description

### Technical Field

The present invention relates to the field of chemical synthesis of pharmaceutical compounds, in particular, paracetamol, and refers to chemical reactions that may be carried out via spray-drying to obtain such compounds.

### Background Art

Synthetic organic chemistry is a perpetual wellspring of discovery and synthesis of small molecules, macromolecules, and polymers, all of which are subject to further exploration and application across a broad spectrum of uses. Progress in this field is linked to the development of novel reactions and synthesis of new compounds, but also to the optimization of synthetic protocols and the exploration of new methodologies or fabrication technologies that facilitate these syntheses.

For instance, paracetamol, also known as acetaminophen or acetyl-para-aminophenol (APAP) is one of the most commonly prescribed medicines, most used active pharmaceutical ingredient (API) worldwide and hence with a great global production rate. In fact, it is listed on the WHO List of Essential Medicines. Not only is it thus important to ensure viability of this drug's supply to all countries, but moreover, new environmentally friendly, as well time- and cost-effective synthetic approaches are needed.

The preparation of paracetamol generally goes through an acylation reaction to form the amide bond (see for instance Parveen, I., et al., in "Two-Step Synthesis of Paracetamol (Acetaminophen), a Practical Illustration of Carbonyl Reactivity for Year-One Biosciences Students", J. Chem. Educ. 2023, 100, 3955-3959).

On the other hand, optimization methods have gained significance in the research area. State of the art documents describe different methods that distance from traditional stir tanks and bulk reactors to conduct, in particular, paracetamol synthesis, by simultaneous hydrogenation and acetylation in one pot, which is reported to increase paracetamol selectivity by avoiding undesirable side reactions of 4-aminophenol (Park, J., et al., "One-pot mechanochemical hydrogenation and acetylation of 4-nitrophenol to 4-aminophenol and paracetamol", Green Chem., 2024, 26, 4079). Notwithstanding, the method disclosed in this document still lacks optimization, particularly for industrial scale-up. The method herein disclosed flows hydrogen gas through a bubbler at 140 °C which is filled with acetic anhydride gas to a ball-milling reactor which contains the reactants, a palladium catalyst, and isopropanol as a solvent. Disadvantages related to this method include safety issues regarding hydrogen gas and acetic anhydride auto-ignition, leaks, the use and recycling of the palladium catalyst, which is associated with a loss in mols of reactant due to adsorption, a reduction in the selectivity of product formation when feedstock is increased, side-products formation, caking due to impact forces generated by ball-milling, and high energy expenditure.

Thus, from what is known in the art, it is derived that there is still the need of simple, safe and cost-effective methods to perform acylation reactions such as to prepare paracetamol in a short period of time and with high conversion rates, yield and purity.

### Summary of Invention

The inventors have surprisingly found that the simultaneous injection of a solution containing reactants (either suspended or dissolved) to perform an amine acylation to yield paracetamol and a gas stream heated at a specific temperature through a two-fluid nozzle into a drying chamber (being this the principle of spray-drying) generates microdroplets that highly accelerate the reaction and displace the equilibrium to products (generally with no need of a purification step) when compared to traditional synthetic methods. This process may be used to conduct any other amine acylation to prepare other amide compounds.

Accordingly, the present invention relates to a process for preparing paracetamol which comprises performing an amine acylation reaction via spray-drying. Amine acylation reactions are pivotal in medicinal chemistry, for instance in the synthesis of paracetamol, as well as in organic chemistry in general, since they are also at the core of generating amine protecting groups.

The removal of solvent and by-products, as well as the effects of confining the reaction within microdroplets greatly accelerate the reactions, obtaining excellent yields in significantly shorter times than those required in bulk conditions. Moreover, these reactions performed in SD, as opposed to the same reactions performed in bulk quantities, avoid the use of constant energy supply, since the generation of molecules occurs spontaneously and also bypass the need of work-up protocols, such as purification steps. Minimal solvent use, followed by its evaporation and recycling does not only enable working in continuous mode, but further reduces the amount of waste generated by this synthetic method. For these reasons, the method of the present invention is considered suitable for synthetic organic chemistry at industrial level, in particular, for green industrial-scale applicability, which is gaining ever-more importance in this field.

Reaction conditions provided by SD moreover increase chemoselectivity of the reaction. The inventors have surprisingly found that chemoselective N-acylation of the amine is achieved in the reactions, without detecting the formation of the O-acylation derivative by ¹H NMR analysis. This further represents a great advantage regarding conversion rates and hence purity and yield of the final product, when compared to other synthetic approaches such as traditional bulk methods, which furthermore need purification steps.

Hence, the use of SD as a large-scale microdroplet chemistry platform seems to be a promising path, continuing to push the boundaries between lab- and industrial- scale and synthetic chemistry. For these reasons, the methodology of the present invention supposes an optimization of synthetic protocols, in particular, for small-molecule synthesis, such as paracetamol.

The present invention is thus safer than methods disclosed in the closest prior art, since nitrogen gas instead of hydrogen gas is used and acetic anhydride is in liquid form, hence auto-ignition is discarded. The method of the present invention is simpler, avoiding the use of a bubbler, catalysts and ball-milling reactors which generate impact forces to mix the reagents. As mentioned before, by evaporating the solvent and by-products formed, no side reactions take place and selectivity is increased. In terms of green-chemistry and industrial applicability, the method of the present invention represents an advantage with regards to what is known in the art, due to its simplicity, lower energy expenditure and solvent use, as well as significantly shorter reaction times.

The inventors have thus found that spray-drying (SD) is a method for organic synthesis of small molecules that is easy to use, scalable and commercially available, specifically for the preparation of amide compounds. As far as the inventors know, spray-drying (SD) is widely used in the field of drug formulation to dry and concentrate liquids, to crystallize active species, to precipitate them from solutions in an amorphous state, to form smaller particles, such as nanoparticles or microspheres, to polymerize, to encapsulate, for taste masking, to prepare controlled-release systems, among others, but for other purpose such as co-spray-drying of APIs (active pharmaceutical ingredients) and other substances, or conducting reactions little has been disclosed.

### Brief Description of Drawings

FIG. 1 shows qNMR of Paracetamol synthesized by SD, where TMB is 1,3,5-trimethoxybenzene and SM is 4-aminophenol
FIG. 2 shows Field-Emission Scanning Electron Microscopy image of Paracetamol synthesized by SD.

### Detailed description of the invention

All terms as used herein in this application, unless otherwise stated, shall be understood in their ordinary meaning as known in the art. Other more specific definitions for certain terms as used in the present application are as set forth below and are intended to apply throughout the description and claims.

As used herein, the indefinite articles "a" and "an" are synonymous with "at least one" or "one or more." Unless indicated otherwise, definite articles used herein, such as "the," also include the plural of the noun.

The word "comprise" for the purposes of the present invention encompasses the case of "consisting essentially of" and "consisting of".

Within the context of this specification, "spray-drying" is defined as fine spraying a liquid into a hot gas stream, evaporating and drying the droplets in-flight and separating and collecting the solid particles from the gas stream.

Within the context of this specification, "acylation of amines" is defined as a class of chemical reactions in which an acyl group (R-C=O) is added to a substrate, in this case an amine compound. The compound providing the acyl group is known as the acylating agent, which in this case is an anhydride compound.

For the purpose of the invention, a "microdroplet" or "aerosol", which are synonymous in the context of the application and may be used interchangeably, is defined as emissions which are typically within the range of 1-50 µm in diameter. During the spray-drying process, microdroplets formed have their moisture removed by evaporation to form a dry-powdered product, which may be thereafter damaged by excessive heat.

For the purpose of the invention, a "propeller gas" or "gas stream", which are synonymous in the context of the application and may be used interchangeably, is defined as the gas which comes into contact with and suspends the droplets generated by atomization of the solution containing the reactants.

The expression "feed rate" refers to the rate at which the solution containing the reactants is introduced into the two-fluid nozzle for its atomization.

The expression "flow rate" refers to the rate at which the gas is pumped through the two-fluid nozzle.

Within the context of this specification, "inlet temperature" is the designated temperature at which the gas stream is heated. As defined above, the gas stream comes in contact with the formed droplets and evaporation of the solvent is thus initiated. The temperature of the gas decreases rapidly due to the intensive evaporative cooling while the droplet temperature remains low and thermally sensitive products can be processed. The droplets and particles movement follows the gas streamlines inside the dryer.

As mentioned above, the present invention relates to a process for preparing paracetamol which comprises performing an amine acylation reaction via spray-drying. In particular, the process goes with high yields and purity, in significantly shorter times, and with less solvent and waste generation than traditional bulk methods used up to date.

This aspect may also be formulated as the use of spray-drying to perform amine acylation reactions to prepare paracetamol.

All the particular embodiments of the process to prepare paracetamol by performing an amine acylation reaction via spray-drying are also embodiments of the use of the invention.

Acylation of amines to obtain amides is among the most utilized reactions in the medicinal chemistry field. From the synthesis of small molecules to proteins and polymer chemistry, controlling the assembly of amide bonds plays an indispensable role across several fields.

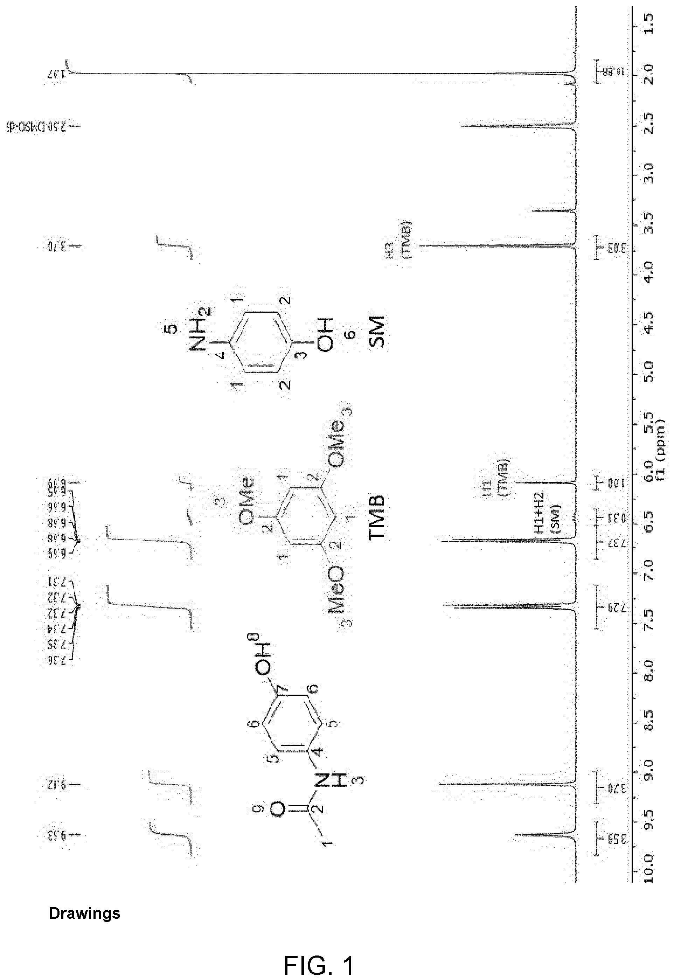

Paracetamol (N-acetyl-para-aminophenol) is one of the WHO's List of Essential Medicines and a molecule widely used as a reaction intermediate, underpinning its significance within the chemical industry. Moreover, its synthesis by spray-drying surprisingly revealed N-acylation selectivity, hence avoiding O-acylation competition issues and obtaining high conversion rates without need for further steps. Surprisingly, no O-acylation product was detected by qNMR (quantitative Nuclear Magnetic Resonance) analysis (see FIG. 1).

It is particularly surprising and advantageous that the inventors have successfully synthesized paracetamol using spray drying with a very high purity and yield. In addition to the possibility of synthesizing in a continuous way and extremely reducing reaction times compared to traditional methods, it is highly advantageous that no work-up steps are needed to obtain the product with high purity, since the by-product, which is acetic acid, is volatile and thus directly removed with the solvent. Moreover, the reaction equilibrium is further displaced to products in that water is concurrently removed during the spraying process. Thus, in a particular embodiment of the process according to the invention, in combination with any of the embodiments above or below, no work-up steps are needed.

In a particular embodiment of the process according to the invention, in combination with any of the embodiments above or below, wherein the acylation reaction comprises reacting an amine compound with an anhydride compound, in the presence of one or more appropriate solvents.

In another particular embodiment of the process according to the invention, in combination with any of the embodiments above or below, the anhydride compound is selected from acetic anhydride and di-tert-butyl dicarbonate.

As can be seen in the Examples, the inventors have also successfully pursued the introduction of protecting groups onto -NH₂ groups by spray drying, in particular, amine protection after reacting 4-aminophenol with excess Boc₂O, easing the process and minimizing need for purification.

In a particular embodiment of the process according to the invention, in combination with any of the embodiments above or below, the amine compound is a primary amine. In a more particular embodiment of the process according to the invention, in combination with any of the embodiments above or below, the primary amine is a phenylamine which is optionally substituted with one or more substituents selected from the group consisting of hydroxy, alkoxy, and alkyl. In an even more particular embodiment, in combination with any of the embodiments above or below, the primary amine is 4-aminophenol.

Preferably, the primary amine used in the reaction in soluble in the appropriate solvent chosen and has a higher boiling and/or melting point than said solvent. Accordingly, 4-aminophenol is soluble in ethanol and has a higher melting point and is hence the preferred primary amine for pursuing the reactions of the Examples.

In a particular embodiment of the process according to the invention, in combination with any of the embodiments above or below, the amine compound and the anhydride compound as defined above are in a suspension or solution.

In another particular embodiment of the process according to the invention, in combination with any of the embodiments above or below, the amine compound and the anhydride compound in the suspension or solution as defined above are each in a concentration that ranges from 0.1 to 0.5 M. In a more particular embodiment, in combination with any of the embodiments above or below, the reactants as defined above are each in a concentration of 0.2 M.

In another particular embodiment of the process according to the invention, in combination with any of the embodiments above or below, the reaction comprises excess anhydride with respect to amine, in a mol/mol ratio ranging from 1:1 to 1:3 of amine: anhydride. In a more particular embodiment of the process according to the invention, in combination with any of the embodiments above or below, the mol/mol ratio of amine: anhydride ranges from 1:1 to 1:1.5. In a more particular embodiment of the process according to the invention, in combination with any of the embodiments above or below, the mol/mol ratio of amine: anhydride is 1:1 when the reactants are 4-aminophenol and acetic anhydride, respectively, and 1:1.3 when the reactants are 4-aminophenol and di-tert-butyl dicarbonate, respectively. In another particular embodiment of the process according to the invention, in combination with any of the embodiments above or below, no work-up steps are needed when the reaction comprises excess anhydride with respect to amine.

Spray-drying-based methodologies offer the distinct advantage of displacing any solvent and/or liquid- or gas-phase byproducts, effectively yielding refined products in a dry-powdered form, as long as the product's melting point exceeds the inlet temperature, which is established according to the boiling point of the solvent to be evaporated. Despite their compatibility with SD (spray-drying), hazardous and toxic solvents such as halogenated solvents, N'N-dimethylformamide (DMF) or dimethyl sulfoxide (DMSO) were excluded from the explored reaction conditions to mimic green industrial-scale applicability.

Thus, in a particular embodiment of the process according to the invention, in combination with any of the embodiments above or below, the appropriate solvent is evaporated, and the reactants and product have a fusion, boiling and/or melting point higher than the boiling point of the solvent to be evaporated. In a more particular embodiment of the process according to the invention, in combination with any of the embodiments above or below, the solvent is one or more selected from the group consisting of ethanol, methanol, tetrahydrofuran, triethylamine, methyl tetrahydrofuran, and acetonitrile. In a more particular embodiment, in combination with any of the embodiments above or below, the solvent is ethanol.

In a particular embodiment of the process according to the invention, in combination with any of the embodiments above or below, the reaction via spray-drying is performed at a constant feed rate, and with a propeller gas at a constant gas flow rate. In a more particular embodiment of the process according to the invention, in combination with any of the embodiments above or below, the propeller gas flow rate ranges from 320 L/h to 380 L/h. In an even more particular embodiment of the process according to the invention, in combination with any of the embodiments above or below, the feed rate is 357 Uh.

In another particular embodiment of the process according to the invention, in combination with any of the embodiments above or below, the feed rate ranges from 0.15 L/h to 0.21 L/h. In a more particular embodiment of the process according to the invention, in combination with any of the embodiments above or below, the propeller gas flow rate is 0.18 L/h. However, at industrial scale, the feed rate could be of, for example, 100 L/h.

In another particular embodiment of the process according to the invention, in combination with any of the embodiments above or below, the propeller gas is flowed at a specific temperature ranging from 70 to 150 °C. In a more particular embodiment of the process according to the invention, in combination with any of the embodiments above or below, the propeller gas is flowed at a temperature ranging from 100 to 130 °C. In an even more particular embodiment of the process according to the invention, in combination with any of the embodiments above or below, the propeller gas is nitrogen.

In another particular embodiment of the process according to the invention, in combination with any of the embodiments above or below, a further work-up and/or purification step may be comprised.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Abbreviations

¹HNMR (Proton Nuclear Magnetic Resonance), WHO (World Health Organization), THF (tetrahydrofuran), MOFs (metal-organic frameworks), ESI (electro-spray ionization), TEA (triethylamine), Boc₂O (di-tert-butyl dicarbonate), SD (Spray-Dryer or spray-drying), DMF (N'N-dimethylformamide), DMSO (dimethyl sulfoxide), TMB (1,3,5-trimethoxybenzene), qNMR (quantitative Nuclear Magnetic Resonance)

### Device and spray-drying conditions used in the Examples

The spray-dryer used to perform the reactions of the Examples is Mini Spray Dryer B-290. In all tested organic reactions, the spray-drying process starts with the atomization of a solution containing the reactants into a spray of microdroplets, facilitated by a two-fluid nozzle . This initial step involves the simultaneous injection of the solution, namely 25 mL of reaction mixture containing substrates at a concentration of 0.2 M, into the Spray-Dryer at a specified feed rate, and nitrogen gas, at another specified flow rate. Both flow and feed rates were maintained at constant values of 357 L/h and 0.18 L/h, respectively. The feed is combined with the propeller gas (nitrogen) inside a nozzle, where each precursor droplet comes into contact with -and is suspended by- the gas stream. The gas stream is heated to a desired temperature (referred to as the inlet temperature, which depends on the solvent's boiling point), in order to evaporate the solvent.

It is then atomized into the drying chamber to form microdroplets. Within the chamber, the microdroplets rapidly evaporate, forming dried microparticles that are directed through a cyclone, separated from the gas stream and collected. Water is concurrently removed during the spraying process. This process leads to the formation of dried microstructured powder, which is then directed through a cyclone, separated from the gas stream, and collected inside a vessel. The obtained crude products were quantitatively analyzed by¹H NMR, and compared to 1,3,5-trimethoxybenzene (TMB) as a quantification standard to determine conversion and purity rates (FIG. 1). Values were then compared with those found on bulk synthesis literature, as well as a direct comparison of reaction times, and solvent consumption volumes, including those of further purification steps.

The present inventors selected 4-aminophenol as the amine substrate, owing to its high melting point and its solubility in ethanol, the chosen reaction solvent for most of the reactions described in the following Examples.

### Examples

### Example 1: Synthesis of paracetamol (N-acetyl-para-aminophenol).

A solution containing 4-aminophenol and acetic anhydride (molar ratio: 1) in 25 mL of THF (tetrahydrofuran) was spray-dried at an inlet temperature of 100 °C for 8.33 min, as shown in the following scheme:

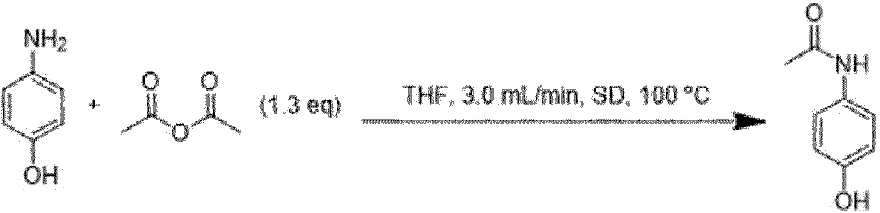

Afterwards, a solid was collected from the spray-dryer, which ¹H NMR confirmed the formation of paracetamol with a 96 % purity and an 87.5 % yield. Formation of particles too small to be separated in the cyclone can explain the difference between substrate conversion and obtained yield. Besides the possibility to synthesize paracetamol in a continuous way and reduce the reactions times, spray-drying also allows to make this drug with high purity without the need of work-up steps because the by-product of this reaction, the acetic acid, is volatile and therefore, it is directly removed with the solvent. Another interesting observation is that this reaction via spray-drying provides a chemoselective N-acylation of 4-aminophenol, without detecting the formation of the O-acylation derivative by ¹H NMR analysis.

The inventors have moreover surprisingly noticed that employing a slight excess of anhydride facilitates the production of paracetamol via spray-drying in excellent purity (98%) and yield (90%).

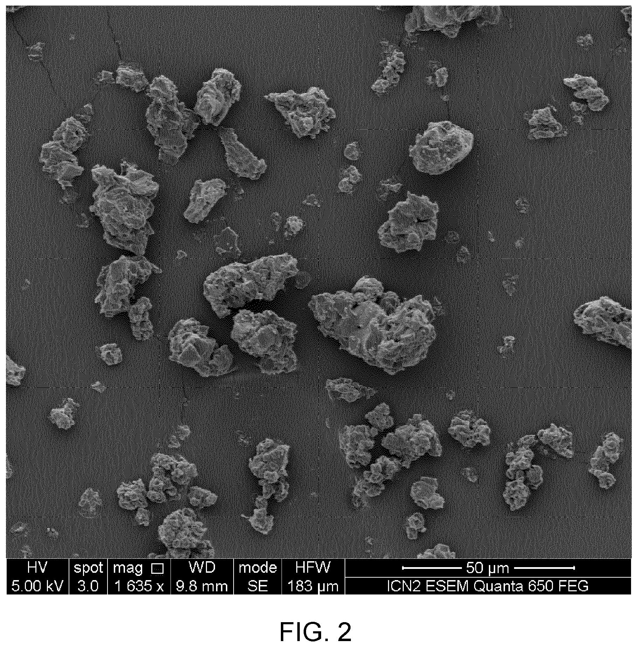

FIG. 1 shows qNMR of paracetamol synthesized by SD, where TMB is 1,3,5-trimethoxybenzene and SM is p-aminophenol, and FIG. 2 shows Field-Emission Scanning Electron Microscopy image of Paracetamol synthesized by SD.

### Citation List

### Patent Literature

- WO2010081236A1

### Non-Patent Literature

- Park, J., et al., "One-pot mechanochemical hydrogenation and acetylation of 4-nitrophenol to 4-aminophenol and paracetamol", Green Chem., 2024, 26, 4079.
- Parveen, I., et al., in "Two-Step Synthesis of Paracetamol (Acetaminophen), a Practical Illustration of Carbonyl Reactivity for Year-One Biosciences Students", J. Chem. Educ. 2023, 100, 3955-3959.

## Claims

1. A process for preparing paracetamol, which comprises performing an amine acylation reaction via spray-drying.

2. The process according to claim 1, wherein the acylation reaction comprises reacting an amine compound with an anhydride compound, in the presence of one or more appropriate solvents.

3. The process according to claim 2, wherein the anhydride compound is selected from acetic anhydride and di-tert-butyl dicarbonate.

4. The process according to claim 2, wherein the amine compound is a primary amine.

5. The process according to claim 4, wherein the primary amine is a phenylamine which is optionally substituted with one or more substituents selected from the group consisting of hydroxy, alkoxy, and alkyl.

6. The process according to any of the claims 2-5, wherein the amine compound and the anhydride compound are in a suspension or solution.

7. The process according to claim 6, wherein the amine compound and the anhydride compound in the suspension or solution are each in a concentration that ranges from 0.1 to 0.5 M.

8. The process according to any of the claims 2-7, wherein the reaction comprises excess anhydride with respect to amine, in a mol/mol ratio ranging from 1:1 to 1:3 of amine: anhydride.

9. The process according to claim 8, wherein the mol/mol ratio of amine: anhydride is 1:1 when the reactants are 4-aminophenol and acetic anhydride, respectively, and 1:1.3 when the reactants are 4-aminophenol and di-tert-butyl dicarbonate, respectively.

10. The process according to any of the claims 1-9, wherein the process comprises evaporating the appropriate solvent, and the reactants and product have a fusion, boiling and/or melting point higher than the boiling point of the solvent to be evaporated.

11. The process according to claim 10, wherein the solvent is one or more selected from the group consisting of ethanol, methanol, tetrahydrofuran, triethylamine, methyl tetrahydrofuran, and acetonitrile.

12. The process according to any of the claims 1-11, wherein the reaction via spray-drying is performed at a constant feed rate, and with a propeller gas at a constant gas flow rate.

13. The process according to claim 12, wherein the propeller gas flow rate ranges from 320 L/h to 380 L/h.

14. The process according to any of the claims 12-13, wherein the gas is flowed at a specific temperature ranging from 70 to 150 °C.

15. The process according to any of the claims 12-14, wherein the propeller gas is nitrogen.
